(19) Europäisches Patentamt
European Patent Office
Office européen des brevets

(11) Veröffentlichungsnummer: **0 004 016**
**B1**

(12) # EUROPÄISCHE PATENTSCHRIFT

(45) Veröffentlichungstag der Patentschrift: **29.07.81**

(51) Int. Cl.³: **C 07 C 69/67**, C 07 C 67/30

(21) Anmeldenummer: **79100568.9**

(22) Anmeldetag: **26.02.79**

(54) **Verfahren zur Herstellung von Brenztraubensäurealkylestern.**

(30) Priorität: **04.03.78 DE 2809421**

(43) Veröffentlichungstag der Anmeldung:
**19.09.79 Patentblatt 79/19**

(45) Bekanntmachung des Hinweises auf die Patenterteilung:
**29.07.81 Patentblatt 81/30**

(84) Benannte Vertragsstaaten:
**BE CH DE FR GB NL**

(56) Entgegenhaltungen:
**US - A - 1 627 091**

(73) Patentinhaber: **BASF Aktiengesellschaft**
**Carl-Bosch-Strasse 38**
**D-6700 Ludwigshafen (DE)**

(72) Erfinder: **Dudeck, Christian, Dr.**
**Odenwaldring 20**
**D-6703 Limburgerhof (DE)**
Erfinder: **Lehmann, Gunter**
**Bayernstrasse 58**
**D-6700 Ludwigshafen (DE)**
Erfinder: **Meissner, Bernd, Dr.**
**Dammweg 15**
**D-6900 Heidelberg 1 (DE)**
Erfinder: **Diem, Hans, Dr.**
**Feldbergstrasse 63**
**D-6800 Mannheim 25 (DE)**
Erfinder: **Fliege, Werner, Dr.**
**Authariestrasse 6**
**D-6701 Otterstadt (DE)**
Erfinder: **Petri, Norbert, Dr.**
**Max-Beckmann-Strasse 17**
**D-6710 Frankenthal (DE)**
Erfinder: **Ross, Karl-Heinz, Dr.**
**Sudetenstrasse 8**
**D-6704 Mutterstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 004 016 B1

## Verfahren zur Herstellung von Brenztraubensäurealkylestern

Die Erfindung betrifft ein neues Verfahren zur Herstellung von Brenztraubensäurealkylestern durch Oxidation von Milchsäurealkylestern in Gegenwart eines Silberkatalysators bestimmter Korngröße bei 450 bis 700°C.

Die DRP 447 838 beschreibt ein Verfahren, worin Oxosäureester aus den entsprechenden Oxysäureestern durch Umsetzung mit Luft in der Gasphase an Katalysatoren hergestellt werden. Die Katalysatoren sind Oxide von saurem Charakter, die sich von Elementen mit mehreren Oxidationsstufen ableiten, oder Metallsalze der sich von diesen Oxiden ableitenden Säuren. Der Katalysator kann auch auf Trägern wie Kupfer, aufgebracht werden. Die Reaktionstemperatur beträgt 200 bis 400°C. Ausbeuten von 60 bis 70 Prozent der Theorie werden beschrieben. Im Falle der Oxidation von Milchsäurealkylestern zeigen die Beispiele nur Vanadiumpentoxid als Katalysator. Silbervanadat wird nur für die Oxidation von Glykolsäureäthylester verwendet. Nachteilig ist, daß statt mit Luft mit Sauerstoff im Überschuß oxidiert wird und Reaktionsrohre von 10 Meter Länge verwendet werden müssen.

In Beispiel 4 wird beschrieben, daß das Endprodukt noch wenige Prozente unveränderten Milchsäureester enthält. Nun ist aber aus Can. J. Res., Band 24, Seite 221 (1946) bekannt, daß Mischungen aus Milchsäureäthylester und Brenztraubensäureäthylester durch fraktionierte Destillation schwierig zu trennen sind, so daß die Gewinnung der Ester in reiner Form bei diesem Verfahren nur durch hohen technischen Aufwand zu erreichen ist.

Um die schwierige Trennung von Endprodukt und Ausgangsprodukt zu verhindern, benötigt man nach Can. J. Res. (loc. cit.), Seiten 221 bis 223, einen Katalysator, der bei der Umsetzung von Milchsäureäthylester zu Brenztraubensäureäthylester vollständigen Umsatz bewirkt. An einem mit Platinmohr elektrolytisch beschichteten Platinnetz wird bei gutem Umsatz eine Ausbeute von 68 Prozent erreicht. Der Katalysator ist unwirtschaftlich, aufwendig in der Regenerierung und hat nur eine Lebensdauer von ca. 45 Stunden. Die Raum-Zeit-Ausbeute beträgt nur 0,085 Gramm pro Kubikzentimeter Katalysatorvolumen und Stunde. Es wird darauf hingewiesen, daß verschiedene Metallkatalysatoren, darunter auch Silber auf Kupferdrahtnetz, in ihrer Wirkung ungenügend sind; bei 250 bis 300°C erreicht man mit ihnen nur 35 bis 50 Prozent Umsatz.

Im Hinblick auf den Stand der Technik weist Ullmanns Encyklopädie der technischen Chemie, Band 4, Seite 727 darauf hin, daß trotz verschiedener synthetischer Bildungsmöglichkeiten das Erhitzen von Weinsäure mit Kaliumbisulfat die beste Darstellung von Brenztraubensäure bleibt. Eine Ausbeute von 50 bis 55 Prozent der Theorie wird bei diesen Verfahren beschrieben.

Alle diese Verfahren sind im Hinblick auf Einfachheit und Wirtschaftlichkeit des Betriebs, Ausbeute und Raum-Zeit-Ausbeute zusammen mit einfacher Aufarbeitung und Reinheit des Endstoffes unbefriedigend.

Es wurde nun gefunden, daß man Brenztraubensäurealkylester der Formel.

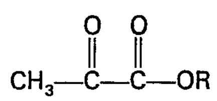

I,

worin R einen aliphatischen Rest bedeutet, durch Oxidation von Milchsäureestern in Gegenwart eines Metallkatalysators bei erhöhter Temperatur vorteilhaft erhält, wenn man Milchsäurealkylester der Formel

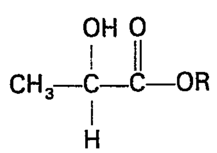

II,

worin R die vorgenannte Bedeutung besitzt, in Gegenwart von Silberkristallen mit einer Korngröße von 0,01 Mikrometer bis 2,5 Millimetern als Katalysator bei einer Temperatur von 450 bis 700°C oxidiert.

Weiterhin wurde gefunden, daß man das Verfahren vorteilhaft ausführt, wenn der Katalysator aus einem Anteil von Silverkristallen mit einer Korngröße von 0,01 bis 10 Mikrometern und einem Anteil von Silberkristallen mit einer Korngröße von 0,2 bis 2,5 Millimetern besteht.

Die Umsetzung kann für den Fall der Verwendung von Milchsäureäthylester durch die folgenden Formeln wiedergeben werden:

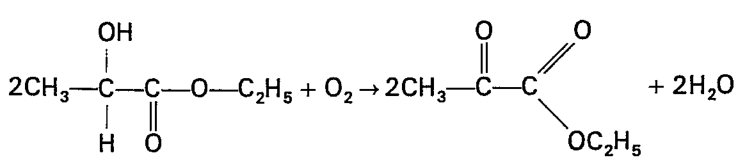

Im Vergleich zum Stand der Technik liefert das Verfahren nach der Erfindung überraschend auf

einfacherem und wirtschaftlicherem Wege ein besseres Gesamtergebnis mit Bezug auf Ausbeute, Raum-Zeit-Ausbeute und Reinheit des Endstoffes sowie Lebensdauer des Katalysators. Die Lebensdauer des Katalysators beträgt in der Regel mindestens 123 Tage im Falle von Brenztraubensäureäthylester. Alle diese vorteilhaften Ergebnisse sind im Hinblick auf den Stand der Technik, insbesondere auf die Lehre in Can. J. Res. (loc. cit.), überraschend, denn man hätte angesichts der hohen erfindungsgemäßen Temperaturen und des erfindungsgemäßen Katalysators zumindest eine wesentliche Verschlechterung der Ausbeute und erhebliche Bildung von Zersetzungsprodukten erwarten sollen. Man erhält im allgemeinen mit den Estern von niederen Alkanolen, insbesondere bei Reaktionstemperaturen von 540 bis 600°C und Katalysatorbelastungen von 0,8 bis 1 $t/m^2 . h$ und bei einem Umsatz von 96 bis 99 Prozent Ausbeuten von 65 bis 75% der Theorie und Raum-Zeit-Ausbeuten von 10 bis 24 $g/cm^3 . h$. Der Brenztraubensäure-n-alkylester kann gegebenenfalls zu Brenztraubensäure verseift werden.

Stört die Anwesenheit geringer Mengen an Ausgangsstoff nicht, z.B. wenn man den Endstoff direkt ohne Reinigung zur Herstellung von Brenztraubensäure verwendet, kann man die Reaktion z.B. bei Temperaturen von 540 bis 600°C und Katalysatorbelastungen von 1,1 bis 1,5 $t/m^2 . h$ durchführen und so bei von 85 bis 90 Prozent Umsatz eine Ausbeute von 80 bis 85 Prozent der Theorie und eine Raum-Zeit-Ausbeute von 25 bis 50 $g/cm^3 . h$ erzielen. Kostspielige Katalysatoraufbereitung bzw. aufwendige Reaktoren werden vermieden.

Der Katalysator ist vergleichsweise leicht regenerierbar. Silberkristalle aller Teilchengrößen, wie sie auch bei der elektrolytischen Herstellung des Silbergranulats anfallen, werden verwendet. Bei dem erfindungsgemäßen Verfahren werden somit die Elektrolysieranlagen besser ausgenutzt und können entsprechend dimensioniert werden; Energie, Betriebspersonal und Hilfsstoffe, z.B. Salpetersäure, werden eingespart und Operationen wie Wäsche, Siebung und Trocknung des Silbers vereinfacht.

Bevorzugte Ausgangsstoffe II und dementsprechend bevorzugte Endstoffe I sind solche, in deren Formeln R einen Alkylrest mit 1 bis 7 Kohlenstoffatomen bedeutet. Der vorgenannte Rest kann noch durch unter den Reaktionsbedingungen inerte Gruppen, z.B. Alkylgruppen mit 1 bis 4 Kohlenstoffatomen, substituiert sein.

Es kommen z.B. als Ausgangsstoffe II in Frage: Der Methyl-, Äthyl-, n-Propyl-, Isopropyl, n-Butyl-, sek.-Butyl-, Isobutyl-, tert.-Butyl-, Pentyl-, Hexyl-, Heptyl-ester der Milchsäure.

Als oxidierendes Agens lassen sich sowohl der reine Sauerstoff als auch freien Sauerstoff enthaltende Gase, insbesondere Luft, verwenden. Sauerstoff, in der Regel in Gestalt von Luft, und Ausgangsstoff II werden zweckmäßig im Molverhältnis von 0,25 bis 0,9, insbesondere von 0,35 bis 0,7 Mol Sauerstoff je Mol Ausgangsstoff II angewandt. Eine Verwendung von Inertgas ist nicht notwendig, stört jedoch andererseits auch nicht die Umsetzung. Gegebenenfalls kann man mit heißen Inertgasen, zwechmäßig Stickstoff oder rußarmen Verbrennungsgasen, die keine Katalysatorgifte enthalten, z.B. von einer Temperatur von 600 bis 800°C, den Katalysator erhitzen.

Für das Verfahren geeignete Ausgangsstoffe sind reiner Milchsäurealkylester oder technischer, d.h. nicht durch eine Reinigungsoperation gereinigter, direkt nach der Herstellung abgetrennter Milchsäurealkylester oder deren Mischungen mit Wasser; die Konzentration der wäßrigen Gemische kann zweckmäßig zwischen 60 und 95 Gewichtsprozent, vorzugsweise zwischen 70 und 90 Gewichtsprozent, schwanken, Zweckmäßig wird für die Reaktion ein Verhältnis von 0,67 bis 0,05 vorzugsweise 0,43 bis 0,11 Gramm Wasser je Gramm Ausgangsstoff II gewählt. Der Milchsäurealkylester wird in Dampfform, gegebenenfalls im Gemisch mit Wasserdampf und gegebenenfalls mit Inertgas, dem Reaktionsraum zugeführt.

Die Gesamtschichtdicke des Katalysators beträgt zweckmäßig 10 bis 50, vorzugsweise 20 bis 30 Millimeter. Man kann alle Korngrößen von 0,01 Mikrometer bis 2,5 Millimeter, vorzugsweise 0,1 Millimeter bis 2,5 Millimeter, im Katalysator verwenden, wobei man zweckmäßig eine homogene Vermischung aller Korngrößen in der Katalysatorschicht wählt. Ebenfalls kann man den Katalysator zwar mit allen vorgenannten Korngrößen bilden, diese aber je nach Korngröße in 2, 3 oder mehr Schichten anordnen. Verwendet man alle Korngrößen im Katalysator, ist ein 2-Schichtkatalysator und insbesondere ein Einschichtkatalysator bevorzugt. Die Katalysatorteilchen in Gestalt von Silberkristallen befinden sich in Katalysatoren mit mehreren Schichten, z.B. im 2- bzw. 3-Schichtkatalysator des üblicherweise vertikal aufgestellten Reaktors je nach Korngröße in einem oberen und unteren (2-Schichtkatalysator) bzw. oberen, mittleren oder unteren Teil (3-Schichtkatalysator) der Gesamtschicht angeordnet. Das Ausgangsgemisch aus Dampf des Ausgangsstoffs II und Sauerstoff bzw. Luft und gegebenenfalls Wasserdampf und Inertgas wird im allgemeinen von oben nach unten geführt, so daß die obere Schicht (obere Schichten) gleichzeitig den dem Ausgangsgemisch zugewandten Teil bedeutet. Bei Reaktoren anderer Bauart oder anderer Führung des Ausgangsgemisches gelten sinngemäß alle Angaben der Beschreibung über oberen (unteren) Teil des Katalysators für den entsprechenden, dem Ausgangsgemisch (dem abgeführten Reaktionsgemisch) zugewandten Teil, z.B. bei horizontal angeordneten Reaktoren für den vorderen (hinteren) Teil des Katalysators.

Im Falle der Verwendung aller Korngrößen in einem 2-Schichtenkatalysator haben bevorzugt seine obere Silberschicht einen Anteil von 5 bis 30, vorzugsweise von 10 bis 20 Gewichtsprozent und Teilchen einer Korngröße von 0,01 Mikrometer bis 0,75 Millimeter und eine untere Silberschicht einen Anteil von 70 bis 95, vorzugsweise von 80 bis 90 Gewichtsprozent und Teilchen einer Korngröße von

0,75 bis 2,5 Millimeter. In einer bevorzugten Ausführungsform verwendet man nicht alle Korngrößen, sondern nur den grobkörnigen Anteil von Silberkristallen mit einer Korngröße von 0,1 bis 2,5 Millimeter. Katalysatoren, deren sämtliche Silberkristalle nur diese gröberen Silberkristalle enthalten, werden im folgenden als grobkörnige Katalysatoren definiert.

Der grobkörnige Anteil (Korngröße von 0,1 bis 2,5 Millimeter) kann in einer Schicht oder in mehreren Schichten angeordnet sein, vorteilhaft in einer Schicht, 2 oder Schichten. Im Einschichtkatalysator wird zweckmäßig der grobkörnige Anteil in homogener Verteilung seiner Katalysatorteilchen durch den ganzen Katalysator hindurch vorliegen. Im 2-Schichtkatalysator haben bevorzugt seine obere Silberschicht eine Anteil von 5 bis 30, vorzugsweise 10 bis 20 Gewichtsprozent und Teilchen einer Korngröße von 0,1 bis 0,75 Millimeter und seine untere Silberschicht einen Anteil von 70 bis 95, vorzugsweise von 80 bis 90 Gewichtsprozent und Teilchen einer Korngröße von 0,75 bis 2,5 Millimeter.

Ist der grobkörnige Anteil in 3 Schichten angeordnet, so befinden sich vorteilhaft im unteren Teil 72,5 bis 89, vorzugsweise 77,5 bis 82,5 Gewichtsprozent aller Katalysatorteilchen im mittleren Teil 2,5 bis 7,5, vorzugsweise 4,5 bis 6,5 Gewichtsprozent aller Katalysatorteilchen, im oberen Teil 8,5 bis 20, vorzugsweise 13 bis 16 Gewichtsprozent aller Katalysatorteilchen. Die Teilchen des unteren Schichtteils haben vorteilhaft Korngrößen von 1 bis 2,5, die des mittleren Schichtteils von 0,75 bis 1, die des oberen Schichtteils 0,1 bis 0,75 Millimeter. Jeder Schichtteil (grobkörniger Anteil) kann aus einer oder mehreren Schichten, vorzugsweise aus 1 oder 2 Schichten bestehen. Bevorzugt ist ein 2- bis 4-Schichtenkatalysator (grobkörnig). Jede dieser Schichten unterscheidet sich von der anderen in der Korngröße der Silberkristalle und meistens auch im zugehörigen Gewichtsanteil des Gesamtkatalysators (grobkörnig).

Hat der obere Schichtteil 2 Schichten, so haben seine untere Schicht bevorzugt einen Anteil von 0,5 bis 2 Gewichtsprozent und Teilchen einer Korngröße von 0,1 bis 0,4 Millimeter und seine obere Schicht entsprechend einen Gewichtsanteil von 8 bis 18 Gewichtsprozent und Teilchen der Korngröße von 0,4 bis 0,75 Millimeter. Entsprechend sind bei dem mittleren Schichtteil mit Bezug auf Gewichtsanteil (Korngröße der Teilchen) bevorzugt:

2 Schichten: obere Schicht 1,5 bis 4,5 (0,75 bis 0,9 mm) Gew.%;
untere Schicht 1 bis 3 (0,9 bis 1 mm) Gew.%.

Bei dem unteren Schichtteil sind bevorzugt:

2 Schichten: obere Schicht 7,5 bis 22,5 (1 bis 1,75 mm) Gew.%;
untere Schicht 50 bis 81,5 (1 bis 2,5 mm) Gew.%.

Das feinkörnige Silber (Korngröße 0,01 bis 10$\mu$m) kann nach bekannten Methoden, z.B. bei der Silbergewinnung mit entsprechenden Mahloperationen und Sieboperationen oder in Gestalt von Raney-Silber hergestellt werden. Bezüglich der Herstellungsmethoden von Silber wird auf Ullmanns Encyklopädie der technischen Chemie, Band 15, Seiten 636 bis 666, verwiesen. Ebenfalls kann Silber aus entsprechenden Lösungen, z.B. Silbernitratlösungen mit Fällungsmitteln, z.B. mit Hydrazin oder Formaldehyd, ausgefällt oder durch Elektrolyse gewonnen werden.

Zweckmäßig belastet man den Katalysator mit 0,5 bis 3 t, insbesondere 0,8 bis 1,8 t Ausgangsstoff II je $m^2$ Katalysatorbettquerschnitt und Stunde. Zur großtechnischen Ausführung verwendet man bevorzugt Katalysatorbettdurchmesser von mindestens 0,1, zweckmäßig 0,2 bis 3 Meter.

Zur Oxidation leitet man ein Gasgemisch aus dampfförmigem Ausgangsstoff II, Luft, gegebenenfalls Intertgas und gegebenenfalls Wasserdampf in vorgenannten Mengen bei Temperaturen von etwa 450 bis 700°C, insbesondere 540 bis 600°C, durch den Silberkatalysator. Das Verfahren wird im allgemeinen bei Drücken zwischen 0,5 und 2 bar, vorzugsweise zwischen 0,8 und 1,8 bar, kontinuierlich durchgeführt. Es ist dabei vorteilhaft, die die Katalysatorzone verlassenden Reaktionsgase innerhalb kurzer Zeit abzukühlen, z.B. auf Temperaturen von 20°C. Das abgekühlte Gasgemisch wird dann zweckmäßig einem Absorptionsturm zugeführt, in welchem der Endstoff mit Wasser, vorteilhaft im Gegenstrom, aus dem Gasgemisch gewaschen wird.

Die nach dem Verfahren der Erfindung herstellbaren Brenztraubensäurealkylester sind wertvolle Ausgangsstoffe für die Herstellung von Arzneimitteln, Kunstharzen und Kunststoffen. So können sie zu Brenztraubensäure, z.B. mit Wasser in Gegenwart eines Kationenaustauschers bei 80 bis 100°C, verseift werden. Bezüglich der Verwendung wird auf den genannten Band von Ullmann, Seite 727, verwiesen.

Die in den folgenden Beispielen angeführten Teile bedeuten Gewichtsteile.

Beispiel 1

Man verwendet eine Anlage mit Verdampfer und einem senkrechten Rohrreaktor. Der Reaktor enthält an seinem Kopf die Zuführung für das dampfförmige Ausgangsgemisch und die Reaktorhaube. Die Katalysatorschicht liegt unterhalb des Reaktorkopfes, weiter unten folgt eine Kühlzone. Der Reaktor ist mit einer Absorptionskolonne verbunden.

In den Reaktor wird ein Katalysator aus Silberkristallen (187 Teilen) folgender Zusammensetzung eingetragen:

| | Anteil am Katalysator (Gew.%) | Korngröße (mm) |
|---|---|---|
| Schicht 1 | 14,1 | 0,1 bis 0,75 |
| Schicht 2 | 5,9 | 0,75 bis 1 |
| Schicht 3 | 80,0 | 1 bis 2,5 |

Dem Verdampfer wird pro Stunde ein Gemisch von 2 051 Teilen Milchsäureäthylester und 1 220 Teile Luft zugeführt und verdampft. Das dampfförmige Ausgangsgemisch wird durch den Katalysator geleitet und bei 550°C und 1,4 bar umgesetzt. Die Belastung beträgt 0,9 $t/m^2$. h. Das gasförmige Reaktionsgemisch wird nun auf 20°C abgekühlt und anschließend mit Wasser gewaschen. In Form einer 72-gewichtsprozentigen Lösung erhält man 1 371 Teile pro Stunde Brenztraubensäureäthylester vom Kp 155°C, entsprechend einer Ausbeute von 68% der Theorie, und 41 Teile pro Stunde unumgesetzter Ausgangsstoff II. Die Raum-Zeit-Ausbeute beträgt 20 $g/cm^3$ . h. Die Lebensdauer des Katalysators beträgt 123 Tage, der Umsatz beträgt 98 Prozent.

Beispiel 2

Hier wird die gleiche Anlage wie in Beispiel 1 verwendet. In den Reaktor wird ein Katalysator aus Silberkristallen (187 Teilen) folgender Zusammensetzung eingetragen:

| | Anteil am Katalysator (Gew.%) | Korngröße (mm) |
|---|---|---|
| Schicht 1 | 18,5 | 0,1 bis 0,75 |
| Schicht 2 | 81,5 | 0,75 bis 2,5 |

Dem Verdampfer wird pro Stunde ein Gemisch von 2 880 Teilen Milchsäureäthylester und 2 250 Teilen Luft zugeführt und verdampft. Das dampfförmige Ausgangsgemisch wird durch den Katalysator geleitet und bei 550°C und 1,4 bar umgesetzt. Die Belastung beträgt 1,27 $t/m^2$.h. Das gasförmige Reaktionsgemisch wird nun auf 20°C abgekühlt und mit Wasser gewaschen. In Form einer 84-gewichtsprozentigen Lösung erhält man 2 322 Teile pro Stunden Brenztraubensäureäthylester vom Kp 155°C, entsprechend einer Ausbeute von 82% der Theorie, und 31,7 Teile pro Stunde unumgesetzter Ausgangsstoff II. Die Raum-Zeit-Ausbeute beträgt 35 $g/cm^3$ . h. Die Lebensdauer des Katalysators beträgt 130 Tage, der Umsatz beträgt 89 Prozent.

Beispiel 3

Analog Beispiel 2 wird die Umsetzung mit demselben Katalysator; aber mit einer Korngröße von 0,01 Mikrometer bis 0,75 Millimeter in der oberen Silberschicht, durchgeführt. Man erhält dieselben Ergebnisse.

Beispiel 4

Analog Beispiel 1 wird die Umsetzung mit Milchsäuremethylester durchgeführt. Die Belastung beträgt 0,9 $t/m^2$ . h. Man erhält 1 448 Teile Brenztraubensäuremethylester vom Kp 135°C pro Stunde, entsprechend einer Ausbeute von 72% der Theorie, und 20,5 Teile pro Stunde unumgesetzten Ausgangsstoff II. Die Lebensdauer des Katalysators beträgt 123 Tage, der Umsatz beträgt 99 Prozent. Die Raum-Zeit-Ausbeute beträgt 21 $g/cm^3$ . h.

**Patentanspruch**

Verfahren zur Herstellung von Brenztraubensäurealkylestern der Formel

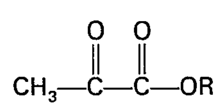

I,

worin R einen aliphatischen Rest bedeutet, durch Oxidation von Milchsäureestern in Gegenwart eines Metallkatalysators bei erhöhter Temperatur, dadurch gekennzeichnet, daß man Milchsäurealkylester der Formel

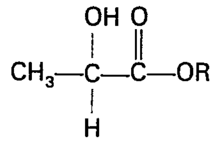 II,

worin R die vorgenannte Bedeutung besitzt, in Gegenwart von Silberkristallen mit einer Korngröße von 0,01 Mikrometer bis 2,5 Millimeter als Katalysator bei einer Temperatur von 450 bis 700°C oxidiert.

**Claim**

A process for the preparation of an alkyl pyruvate of the formula

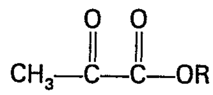 I,

where R is an aliphatic radical, by oxidizing a lactic acid ester in the presence of a metal catalyst at an elevated temperature, characterized in that an alkyl lactate of the formula

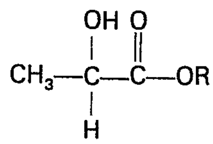 II,

where R has the above meaning, is oxidized in the presence of silver crystals, having a particle size of from 0.01 micrometer to 2.5 millimeters, as the catalyst, at from 450 to 700°C.

**Revendication**

Procédé pour la préparation d'esters alcoyliques d'acide pyruvique de formule

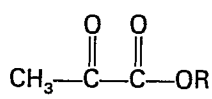 I,

dans laquelle R représente un radical aliphatique, par oxydation d'esters d'acide lactique en présence d'un catalyseur métallique à température élevée, caractérisé en ce qu'on oxyde des esters alcoyliques d'acide lactique de formule

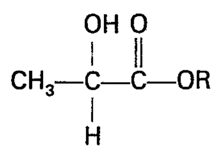 II,

dans laquelle R a la signification donnée ci-dessus, en présence de cristaux d'argent ayant une grosseur de grains de 0,01 microns à 2,5 mm en tant que catalyseur, à une température de 450 à 700°C.